# EUROPÄISCHE PATENTSCHRIFT

(11) **EP 4 405 040 B1**
(45) Veröffentlichungstag und Bekanntmachung des Hinweises auf die Patenterteilung: **11.02.2026**
(21) Anmeldenummer: 22792892.6
(22) Anmeldetag: 26.09.2022
(51) Int. Cl.: A61N 5/10

(54) **MCO PLANUNG VON BEHANDLUNGEN MIT VERFÜGBAREN TECHNOLOGIEN BEI DER RADIOTHERAPIE (RT)**
MCO PLANNING OF TREATMENTS WITH AVAILABLE TECHNOLOGIES IN RADIOTHERAPY (RT)
PLANIFICATION MCO DE TRAITEMENTS AVEC DES TECHNOLOGIES DISPONIBLES EN RADIOTHÉRAPIE (RT)

(30) Priorität: 25.09.2021 DE 102021124814
(43) Veröffentlichungstag der Anmeldung: 31.07.2024
(73) Patentinhaber: Fraunhofer-Gesellschaft zur Förderung der angewandten Forschung e.V., 80686 München (DE)
(72) Erfinder: BORTZ, Michael, 67663 Kaiserslautern (DE); KUEFER, Karl-Heinz, 67685 Weilerbach (DE); SUESS, Philipp, 55257 Budenheim (DE); TEICHERT, Katrin, 67655 Kaiserslautern (DE)
(74) Vertreter: Leonhard, Frank Reimund
(86) Internationale Anmeldenummer: PCT/IB2022/059141
(87) Internationale Veröffentlichungsnummer: WO 2023/047379

(56) Entgegenhaltungen:
- US-A1- 2013 090 549
- US-A1- 2017 189 715
- US-A1- 2019 083 814
- US-A1- 2021 052 916
- US-A1- 2021 205 635
- US-A1- 2021 228 907

## Beschreibung

Diese Offenbarung (und Ansprüche) betreffen ein Verfahren der multikriteriellen Optimierung (MCO).

Ein Krebspatient soll eine Strahlentherapie (Radiotherapie - RT) erhalten. Hierfür ist eine Mindestdosis für das Tumorgewebe vorgesehen und Höchstdosen für umliegende gesunde Gewebestrukturen, die idealerweise nicht ausgeschöpft werden sollten. Prinzipiell kommen für einen solchen Patienten mehrere technische Möglichkeiten (Technologien) in Frage, mit denen eine solche Behandlung durchgeführt werden kann.

Beispielhaft zu nennen sind:
1. Protonen
2. Photonen per Rotationstherapie mit 1 Umdrehung
3. Photonen per Rotationstherapie mit 2 Umdrehungen
4. Photonen per IMRT mit 9 Feldern (mit 9 festgesetzten Winkeln)
5. Photonen per IMRT mit 9 Feldern (mit anderen Winkeln als in Option 4)
6. Moderne Geräte neuer Technologie
7. Technisch ältere Therapiegeräte, die schon länger in Gebrauch sind.

Jede dieser Technologien kann auf einem oder mehreren verschiedenen Therapiegeräten durchgeführt werden. Jedes Gerät ist anders anzusprechen und bedarf einer spezifischen Planung, wobei die individuellen Planziele über die eingesetzten Technologien identisch bleiben. In diesem Zusammenhang wird auf die im Dokument US 2013/090549 A1 offenbarte technische Lehre verwiesen.

Aus Sicht der Klinik relevant ist das Planen aller Behandlungsfälle (Gesamtplanung) unter Berücksichtigung aller zur Verfügung stehender Therapiegeräte. Die Gesamtplanung soll nicht nur unter den individuellen medizinischen Aspekten eine Therapie gerecht werden, sondern gleichzeitig soll eine Terminierung der Behandlungen vorgenommen werden.

Mathematisch entsteht ein mehrkriterielles Planungsproblem mit den Zielen: Hohe Wahrscheinlichkeit eines Therapieerfolgs bei gleichzeitiger Vermeidung von Nebenwirkungen bei jeder individuellen Therapie und möglichst effektive Nutzung der zur Verfügung stehenden Therapiegeräte, mit denen möglichst viele Patienten eine Therapie erhalten können, ohne lange Wartezeiten in Kauf nehmen zu müssen (Time to Treatment).

Stand der Technik ist ein zweistufiger Entscheidungsansatz. Zuerst wird die Technologie festgelegt, dann wird diese Technologie geplant. Dabei können beim zweiten Schritt mehrkriterielle Planungsmethoden zum Einsatz kommen. Die Entscheidung über die Auswahl der Technologie treffen Ärzte aufgrund von Erfahrung und Ergebnissen klinischer Studien. Will man entscheiden, welche Technologie sich für einen Behandlungsfall am besten eignet, muss für jede mögliche Technologie eine separate Planung durchgeführt werden, die jeweiligen Pläne verglichen und dann die "beste" ausgesucht werden.

Der klinische Ablauf ist auch zu bedenken: Für dringende Fälle müssen bereits auf bestimmte Geräte terminierte Behandlungen verschoben oder sogar auf andere Geräten umgeplant werden. Oder der dringend eine Therapie benötigende Patient muss warten.

Jede Technologie muss mit bestimmten, dafür vorgesehenen Geräten erfolgen - eine Behandlung mit Protonen kann nicht mit einem Photonenbeschleuniger erfolgen. Die Anzahl der zur Behandlung verfügbaren Geräte ist einer der limitierenden Faktoren für Krankenhäuser. Mehr Patienten zu behandeln - gerade Protonenanlagen sind auf Grund ihrer Kosten und Komplexität nur sehr begrenzt verfügbar - zwingt zu Wartezeiten oder Therapie-Umplanungen. Erkennbar wird ein mehrkriterielles Optimierungsproblem; möglichst viele Patienten in gegebener Zeit - oder bei gegebenem, meist beschränktem Investitionskapital, behandeln zu können.

Besitzt ein Krankenhaus nun zumindest zwei Geräte, die für eine Behandlung in Frage kommen, kann rein organisatorisch geplant werden (per Scheduling), welche (Tages-)Fraktionen einer fraktionierten Behandlung eines Patienten auf welchem der im Beispiel genannten Strahlengeräte die Behandlung ausgeführt werden soll. Ist eines der Geräte über einen Zeitraum schon belegt von einem anderen Patienten, kann dieses Gerät für diese Planung des neuen Patienten nicht mit einbezogen werden. Der Patient muss entweder eine deutliche Wartezeit in Anspruch nehmen oder abgewiesen werden. Gerade bei der Therapie von bösartigen Tumoren ist es besonders unerwünscht, Patienten mit Hinweis auf belegte Geräte warten zu lassen oder gar abzuweisen. Es kommt hier in besonderem Maße darauf an, die auf den Zeitraum der Fraktionierung vorgesehene Behandlung auch möglichst schnell zu beginnen. Ein bösartiger Tumor ist beispielsweise keine Sache, die einen langen Aufschub erlaubt. Besonders solche Patienten sind Risikopatienten, wobei das Risiko hier die Zeitspanne ist, bis zum Beginn einer Behandlung.

Eine andere Schwierigkeit ist es, technische Strahlengeräte jüngeren Herstellungsdatums ebenso zu benutzen, wie solche technische Strahlengeräte, älteren Datums. Es zeigt sich, dass die modernen, erst kürzlich angeschafften Geräte dauerhaft belastet sind, oder dauerhaft ausgelastet sind, und sich kaum oder keine Patienten finden, die sich mit den schon betagten Geräten behandeln lassen oder ärztlich verordnet behandelt werden.

Das technische Problem der Erfindung liegt darin, jedem Patienten individuell eine gute Therapie zukommen lassen zu können, wobei die Güte der Therapie auch die Minimierung der Wartezeit jedes Patienten einbezieht (unter "time to treatment" ein technisches Kriterium einer Therapieplanung).

Die Erfindung geht von der Erkenntnis aus, dass eine geplante Gesamtdosis einer geplanten Therapie auf mehrere Behandlungs-Sitzungen verteilt wird und dabei die Fraktionen von nicht gleichen Strahlengeräten stammen. Es wird von zwei nicht gleichen Strahlengeräten ausgegangen, und damit von Tagesdosen von einem Strahlengerät und andere Tagesdosen von einem anderen Strahlengerät abgegeben. In einer Zusammenschau der gesamten Therapie entsteht so eine kombinatorische Wirkung durch eine Summe der Dosen pro Tag über die Therapiedauer. Wird von einer "Mischung von Technologien", "gemischten Therapie" oder "kombinierter Strahlenabgabe" gesprochen, ist gemeint, dass eine gewisse Anzahl an (Tages-)Fraktionen der geplanten Therapie (mit einer Gesamtanzahl an Fraktionen) auf einem Gerät und die verbleibende Anzahl an (Tages-)Fraktionen auf einem anderen Gerät verabreicht werden. Hier indes alles im Rahmen der Planung dieser Therapie, nicht der Therapie selber. Hinweise darauf beziehen sich grundsätzlich auf die Planung.

Werden zwei Strahlengeräte, Therapiegerät A und Therapiegerät B, verwendet, gibt es mit dieser Lösung eine solche, bei der nur das Therapiegerät A seine Strahlen auf den Patienten abgibt, dann wäre es eine reine A-Behandlung. Es gibt eine andere Variante, bei der nur das Therapiegerät B seine Strahlen auf den Patienten abgibt, dann wäre es ist eine B-Behandlung.

Jede andere Art von kombinierter Strahlenabgabe von beiden Geräten während der Therapiedauer auf denselben Patienten ist eine kombinatorische Bestrahlung, die in ihrer Wirkung zwischen "nur A" und "nur B" liegt. Um die Planungen dieser Therapien, nicht um deren Anwendung geht es hier.

Auf die Ansprüche wird verwiesen, sie werden hier zur Lösung des genannten Problems einbezogen, insbesondere die unabhängigen Claims 1 und 7.

Ausführungsbeispiele der Erfindung sind mit Hilfe der Figuren näher erläutert. Alle Erläuterungen sind gleichermaßen für die Offenbarung heranzuziehen, aber sie sind nicht so auszulegen, dass sie als notwendige Bestandteile der Ansprüche hinzugenommen werden müssen. Alle folgenden Beispiele bleiben auch dann Beispiele, wenn nicht explizit "beispielsweise" ihnen vorgelagert ist.
- Figur 1: ist eine schematische Ansicht eines Beispiels eines Ablaufs eines Verfahrens zur Planung einer Therapie. Hier mit Patches 401 und 201 als Technologien auf einem Sichtgerät mit Display 10 (repräsentiert mit den vier gestrichelten Ecken) und zwei Bedienhilfen 21, 22 als Slider mit jeweiligem "Handle" 21a und 21b (als grafisch dargestellter Bedienknopf). Der Patch 101 wird als Patch 201 in der folgenden Figur 1A mit seinen Stützstellen dargestellt, die hier für den Patch 101 gelten. Patch 201 entspricht Patch 101.

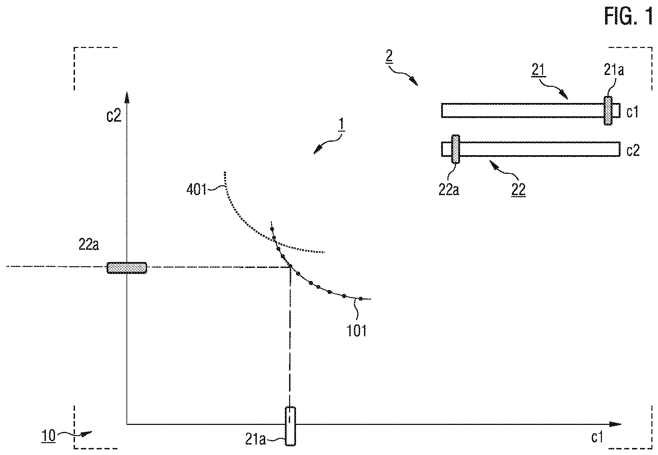

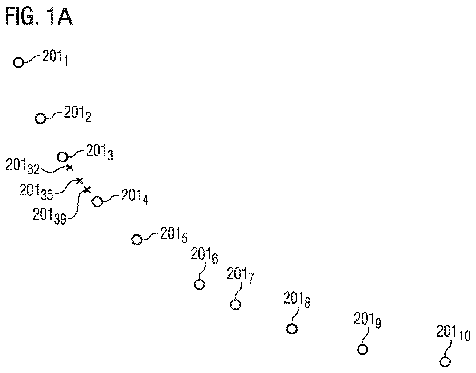

- Figur 1A: hilft bei dem Verständnis der Paretofront, im Beispiel einer Paretofront 201. Erkennbar sind eine Vielzahl von Stützstellen 201₁ bis 201₁₀. Dies ist zu übertragen auf die Paretofront 101 der Figur 1, dort zutreffend als entsprechende Stützstellen 101₁ bis 101₁₀. Die Front 101 ist also keineswegs durchgehend, sondern nur funktional zusammenhängend, daher ist sie in Figur 1 zusätzlich als (dünne) Linie ausgewiesen, die im Grunde keine solche ist. Die Paretofront kann ebenso eine Projektion auf dem n-dimensionalen Raum dargestellt gedacht werden, in welchem Raum sie besteht, indes grafisch nur als eine Projektion dargestellt werden kann.

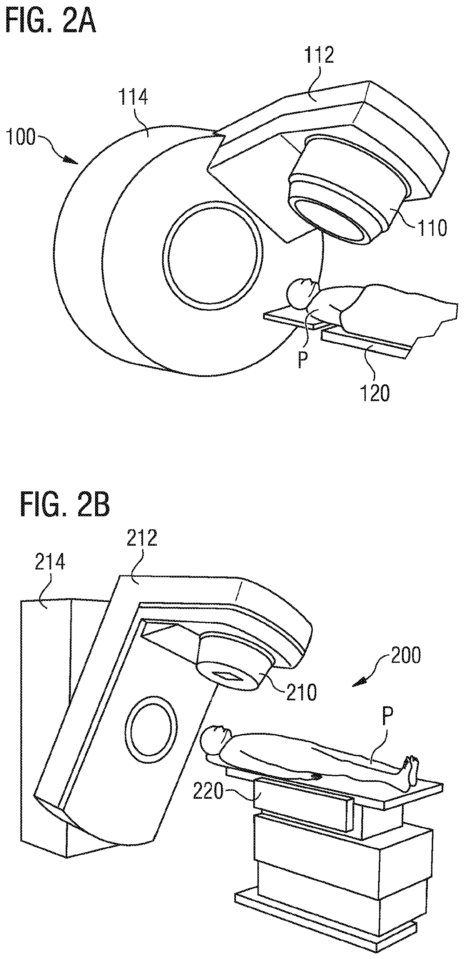

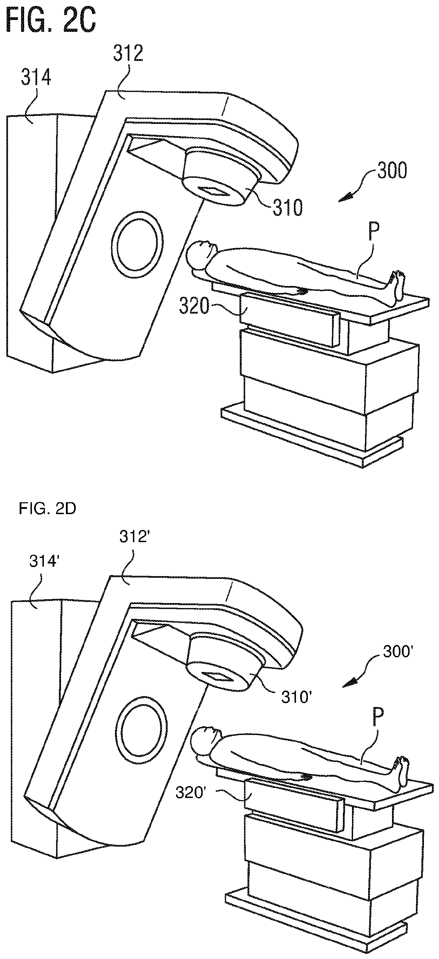

- Figur 2A bis 2D: sind verschiedene Strahlengeräte 100, 200 und 300, 300', die unterschiedliche Strahlengeräte (oder auch "Technologien" bereitstellen. Darunter sind moderne Geräte neuer Technologie und Geräte, die schon länger in Gebrauch sind. Es kann nach Figuren 2C und 2D ein Gerät A der Technologie 300, und ein weiteres Gerät B der Technologie 300' vorhanden sein, das schon älter ist, also weniger gern vom behandelnden Arzt für seine Planung ausgewählt wird, wenn keine anderen Kriterien eine Rolle spielen, z.B. eine sofortige Verfügbarkeit des Strahlengeräts, um zuerst mit dem Strahlengerät nach Figur 2D eine Planung zu beginnen (und damit eine spätere Therapie vorzubereiten). Dies, wenn Strahlengerät A belegt und damit nicht verfügbar ist. Das Strahlengerät der älteren Technologie 300' in Figur 2D soll als eine "andere Technologie" interpretiert werden, obwohl es technisch die gleiche Technologie wäre, wie das moderne Strahlengerät der Figur 2C mit der Technologie 300.
- Figuren 4A bis 4D: zeigen im Ablauf wie ein Feld F entsteht bzw. definiert ist, welches alle Kombinationen an erreichbaren Mischverhältnissen von zwei Technologien mit den Paretofronten 601 und 701 abbildet und dies bei den Zielkriterien c1 und c2.
- Figur 4E: veranschaulicht eine alternative Bildung des Feldes F, wobei die gleichen Punkte auf den Paretofronten verwendet werden, wie in Figur 4B.
- Figuren 5A und 5B: zeigen eine Begrenzung erreichbarer Werte in den Zielkriterien c1 und c2 durch die Intervalle I₁ und I₂ (auch therapeutisches Fenster f genannt), wodurch der Raum an erreichbaren Mischverhältnissen von Technologien eingeschränkt wird, hier dargestellt als beschränktes Feld F'.
- Figur 6: zeigt eine weitere Einschränkung des Feldes F', wobei die Felder F₁" und F₂" entstehen. Die weitere Begrenzung ergibt sich durch das Einstellen eines vorab festgelegten Mindest- (bzw. maximalen) Anteils an Fraktionierungen pro Technologie. Abgebildet wird das Kriterium "mindestens ein 2/3-Anteil der Technologie 601" (bzw. maximal ein 1/3-Anteil der Technologie 701).
- Figur 7: zeigt das Feld F' für den Fall, dass eine Technologie strikt besser ist als eine andere Technologie.
- Figur 8: zeigt das Feld F' für den Fall, dass eine Lücke 1401 im Wertebereich eines der Zielkriterien für zwei ausgewählte Technologien existiert.
- Figur 9: zeigt das Feld F', hier bestehend aus F₁' und F₂', für den Fall, dass sich Synergieeffekte aus der Kombination zweier Technologien ergeben. Das Feld F' wird erweitert.
- Figur 10: zeigen die Paretofronten (mit ihren nicht sichtbaren Stützstellen, die zwischen sich interpoliert werden, sh dazu Figur 1A). Erkennbar sind die Formen der Paretofronten 601 bis 1201 aus den Figuren 4A bis 9. Der aktuell über die Slider angewählte Punkt zₖ₃, wird von den Slidern 21a und 22a im Steuerbereich 2 der Darstellung bestimmt, wobei 1 den funktionell getrennten Patchbereich der Paretofronten bildet.
- Figuren 11A/11B: veranschaulichen zeitliche Verfügbarkeiten von Therapiegeräten A und B (genannt die "TGeräte") in einem Tagesraster von Verfügbarkeiten (hell ist frei, schraffiert ist belegt).
- Figur 12: veranschaulicht ein technisch-funktionelles Datensystem mit Rechner, Speichersystem und dem Eingabe-/Ausgabegerät mit einem Display 10. Das Display 10 kann ein normales Sichtgerät sein, oder ein Touch-Screen, um statt mit einem Mauszeiger mit einem Finger (als Zeigegerät) navigierende Kontrolle über das Planungssystem zu haben.

Die Beschreibung befasst sich damit, dass nicht nur ein Therapiegerät (als Strahlengerät) zur Verfügung steht, sondern mehrere davon. Naturgemäß haben die Therapiegeräte eine Belegung, sind terminlich also von schon vorhandenen Therapien in einem Zeitraster belegt, sodass ein neuer Patient mit einem Therapiebedarf sich in das vorhandene System der Belegungen einpassen muss.

Es führt zu Unmut bis hin zur Angst, wenn ein Patient mit einer kritischen zu behandelnden Krebserkrankung nicht sofort bedient werden kann, also kein Therapieplatz verfügbar ist - und für ihn keine Planung einer Therapie erfolgen kann. Mit anderen Worten ist kein Strahlengerät in dem Augenblick verfügbar, wenn der Patient seinen Therapiebedarf anmeldet, respektive vom Planer zugewiesen erhält.

Eine Möglichkeit, diese frühestmögliche Behandlung durch ihre zugrundeliegende Planung doch zur Verfügung zu stellen, liegt darin, ein anderes Strahlengerät, oder ein Strahlengerät einer anderen Technologie, auch möglich ist damit ein Strahlengerät einer älteren Technologie, in den Therapieplan einzubinden. Es entsteht so eine Mischung aus mehreren Tagesdosen von nicht gleichen Strahlengeräten.

Die "Mischung" soll hier nicht so verstanden werden, dass die Strahlengeräte gemischt werden, sondern dass die Wirkungen der Strahlengeräte auf den therapiebedürftigen Patienten gemischt (oder mit Tagesdosen über den Verlauf der Therapie summiert) werden.

Auch hier soll erneut gesagt werden, dass keine Therapie am Menschen (dem Patient) erfolgt, sondern nur geplant wird. Diese Planung kann außerhalb der Patentansprüche schon als Therapie mit den Strahlengeräten umgesetzt werden, nicht mit den Patentansprüchen. Dennoch kommt es vor, bei der Beschreibung der Planung auch die Strahlentherapie als solches zu erwähnen.

Es soll im Folgenden das Strahlengerät A und das Strahlengerät B, anhand der Figuren sind es deren Paretofunktionen 601 und 701, als die zumindest zwei Strahlengeräte betrachtet werden, deren Wirkungen in der Planung (für die Dauer der fraktionierten Therapie) gemeinsam auf den therapiebedürftigen Patienten einwirken /zutreffend, "als einwirkend angenommen werden"). Mehrere solcher Strahlengeräte sind möglich und können in die Planung eingebunden werden.

Damit dies möglich ist, erfolgt die Mischung in fraktionierten Tagesdosen. Als Zeitraum wird die gesamte Behandlungsdauer betrachtet, und die kleinste Einheit der Mischung ist ein Tag. Diese Aufteilung der Einheiten, Gesamtdauer und diskret der Tag, sind als Beispiel aufzufassen. Sie orientiert sich an der derzeit üblichen fraktionierten Planung für einen Patienten, bei dem ein Tag sich als eine gute, passende Einheit herausgestellt hat, in der ein Patient belastbar ist, sich erholt, und wieder belastbar ist.

Eine Planung der Therapie beginnt mit einem Tag 1 und endet nach der Behandlungsdauer nach dem Tag X, wobei in den Beispielen X mit 30 angegeben ist.

Nach einem Tag Y < X wechselt die Planung auf ein anderes Strahlengerät. Begonnen wird mit dem ersten Strahlengerät.

Für das erste Gerät sollte die Prämisse gelten, "geplanter Therapiebeginn so früh als möglich". Der therapiebedürftige Patient hat damit keine spürbare Wartezeit und bekommt das Gefühl, gleich behandelt werden zu können. Zumeist ist das technisch bestmögliche Strahlengerät oder das Strahlengerät mit der modernsten Technologie terminlich nicht gleich verfügbar, sodass die Wartezeit auf den therapiebedürftigen Patienten störend, unangenehm bis beängstigend wirkt. Eine Therapie anzubieten, die sofort beginnt, wird demgegenüber bevorzugt und selbst wenn das sofort verfügbare Gerät nicht auf dem letzten technischen Stand ist oder nicht die technisch modernste Technologie verfügbar macht, soll dieses Strahlengerät dennoch zu Beginn eingeplant werden. Als Eingangswert könnte eine Terminplanung verwendet werden, da aber auch die Behandlungsqualität eine Rolle spielt, reicht eine bloße Terminplanung nicht aus.

Eine zweite Prämisse, indes keine zwingende Prämisse, ist die Reduzierung der Anzahl der Wechsel zwischen Strahlengeräten. Es wird von dem Patienten als unangenehm empfunden, wenn er - gemäß der Planung - mehrfach das Strahlengerät wechselt oder wechseln soll, also die Planung so ausgerichtet ist, dass ein Wissen über die Verfügbarkeit umgesetzt wird. Andererseits ist sie auf Tagesdosen verteilt, und ein Patient erinnert sich nach einem Tag nicht mehr so genau, auf welchem Strahlengerät er am Vortag seine fraktionierte Tagesdosis erhalten hat.

Maßgeblich ist die Dosisverteilung in den relevanten Voxeln des Gewebes, über den gesamten Zeitraum der geplanten Therapie, hier beschrieben als die zu planende Therapie, da nur diese Planung hier beschrieben wird und auch mit den Ansprüchen beansprucht ist, nicht die am Patienten durchgeführte Strahlentherapie selbst.

Die Kriterien, cᵢ, nach denen eine Therapie geplant wird, sind technischer Natur, zum Beispiel ausgewählt aus den folgenden "mittlere Dosis im Herzen", bestimmte klinische Ziele, Dosis im Target oder Zeitdauer. Die technische Frage die sich stellt, ist, "welche Behandlungsqualität ist möglich", unter der Prämisse "eines frühestmöglichen Beginns". Mathematisch handelt es sich dabei um eine gemischt ganzzahlig nicht konvexe Aufgabenstellung die nur mit den nicht dominierenden Punkten arbeitet (ein Punkt auf der Paretofront entspricht einem Plan mit seinen technischen Einstellgrößen).

Mit der Prämisse, geplante Zeit zum Behandlungsbeginn, Englisch "time to treatment - TTT", wird der Rahmen des Mischungsverhältnisses für die Gesamtdauer der Therapieplanung gesetzt.

Wird mehrfach gewechselt zwischen den Strahlengerät, gemeint ist auch hier die Planung, also geplante Wechsel, hat es auf das sich über die Gesamtdauer ergebende Mischungsverhältnis eine einschränkende Wirkung.

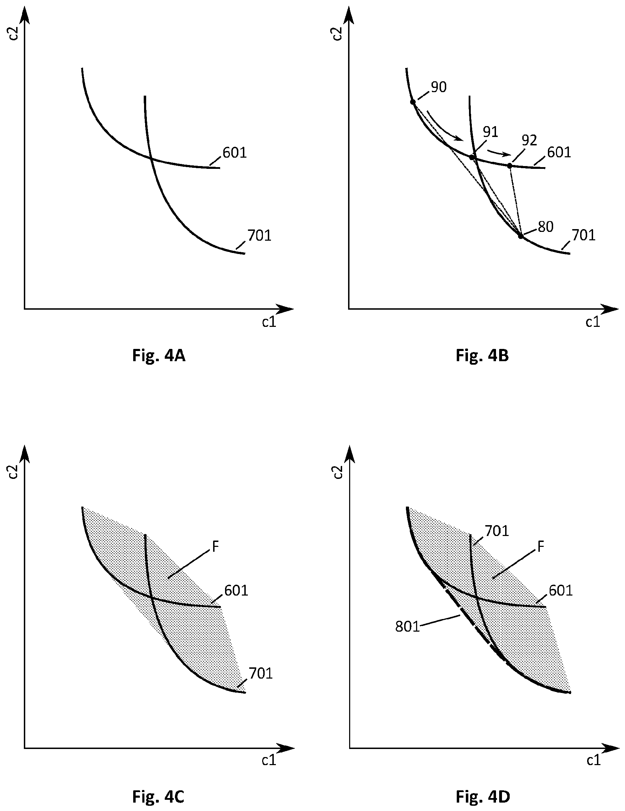

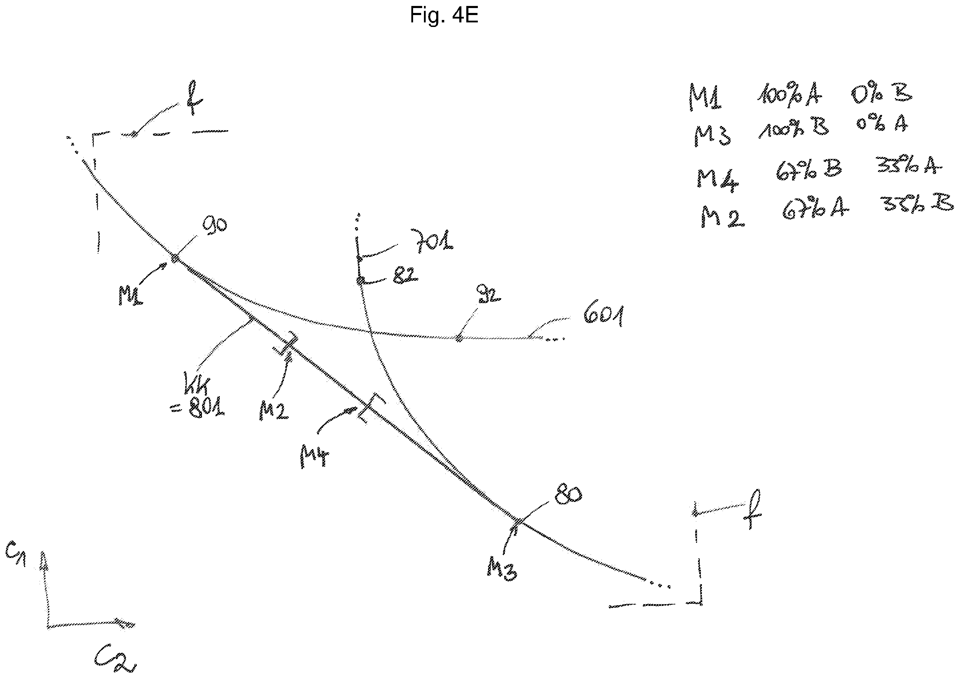

Die Menge F der effizienten Mischungen wird im Rahmen dessen gesehen, was toleriert wird. Es gibt eine Obergrenze, es gibt eine untere Grenze, die ein Mindestmaß an Strahlungsdosis definiert. Es wird dabei ein therapeutisches Fenster aufgespannt, welches in den Figuren mit f benannt ist, sh z.B. Figur 4E. Das therapeutische Fenster kann sich dynamisch verändern oder in seiner Größe variieren.

Vergrößert dargestellt zeigt Figur 1 rechts oben einen Bedienbereich 2 und links, in größerem Flächenverbrauch als der Bedienbereich 2 einen Patchbereich 1. Der Bedienbereich 2 liegt außerhalb des Patchbereichs 1, was sagen soll, dass diese beiden sich nicht funktional behindernd überlappen. Sie erscheinen für den Planer sichtbar voneinander getrennt, wobei ihre Lage nur in dem Beispiel der Figur 1 so gewählt ist, dass der Patchbereich links unten und der Bedienbereich rechts oben (auf der Sichtfläche des Displays 10) angeordnet ist. Andere Anordnungen sind ebenso möglich.

Der Bedienbereich 2 und der Patchbereich 1 sind funktionell miteinander gekoppelt. Diese funktionelle Kopplung soll erläutert werden.

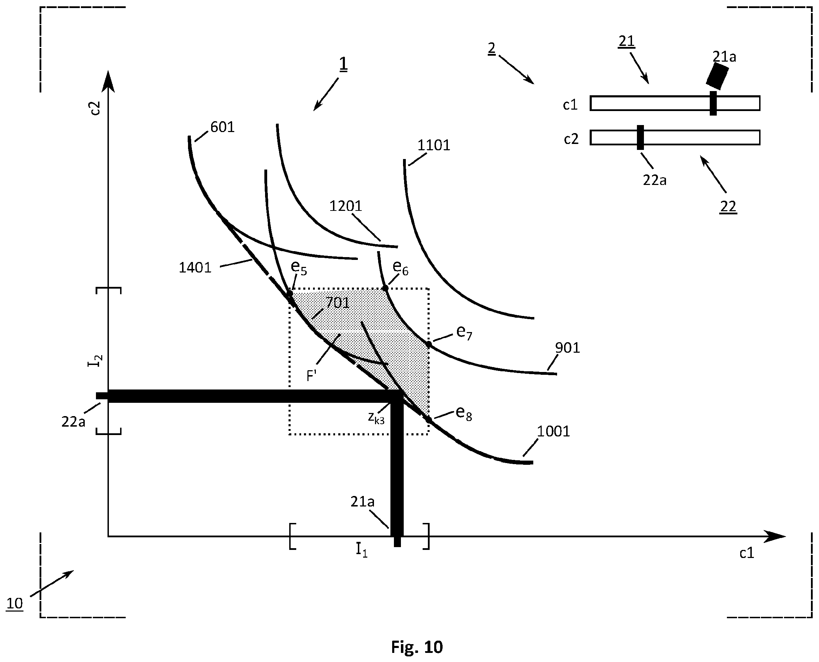

Im Patchbereich 1 werden mehrere Paretofronten gezeigt, im Beispiel sind es die Paretofronten 101 und 401, sowie 601, 701 und weitere in den Figuren 4A bis Figur 10.

Auch dargestellt im Patchbereich 1 sind zwei Achsen c1 und c2 (Kriterium 1 und Kriterium 2), die senkrecht zueinander stehen und zwei Kriterien c1 und c2 repräsentieren. Dargestellt wird das Kriterium 1 (auch c1) und das Kriterium 2 (auch c2), welche im Bedienbereich 2 sichtbar und für den Planer navigierbar dargestellt werden. Die Beschränkung der Darstellung auf hier zwei Kriterien ist nur beispielhaft zu sehen, es werden weit mehr Kriterien sein, die Berücksichtigung finden. Beispiele für mögliche technische Kriterien sind oben genannt. Nachdem in einem darstellbaren kartesischen Raum aber nur drei Kriterien sichtbar dargestellt werden können, keine n Kriterien mit n größer 3, soll es bei diesem Beispiel belassen werden, und der Aussage, dass n Kriterien Berücksichtigung finden, also c1 bis cn. Die Darstellung kann auch eine Projektion aus dem n-dimensionalen Raum sein.

Zum Verständnis der Paretofront(en) soll folgendes angemerkt werden. Jede Paretofront ist eine nur funktionell zusammenhängende Funktion. Jede davon hat ein Vielzahl von Stützstellen (hier 101ᵢ statt der in Figur 1A dargestellten 201ᵢ). Die Front ist also keineswegs als durchgehend zu verstehen, sondern nur "funktional zusammenhängend", daher ist in der Figur 1 zu den Stützstellen auch eine Linie ausgewiesen, die im Grunde keine durchgehende Paretofront darstellt. Sie kann ebenso eine Projektion aus dem n-dimensionalen Raum sein, in welchem Raum sie besteht, indes grafisch nur als eine Projektion dargestellt werden kann. Die Linien repräsentieren die jeweils funktionell zusammenhängende Paretofront. Mit Bezug auf Figur 1A wird das vergrößert verdeutlicht, zwischen den Stützstellen wird interpoliert.

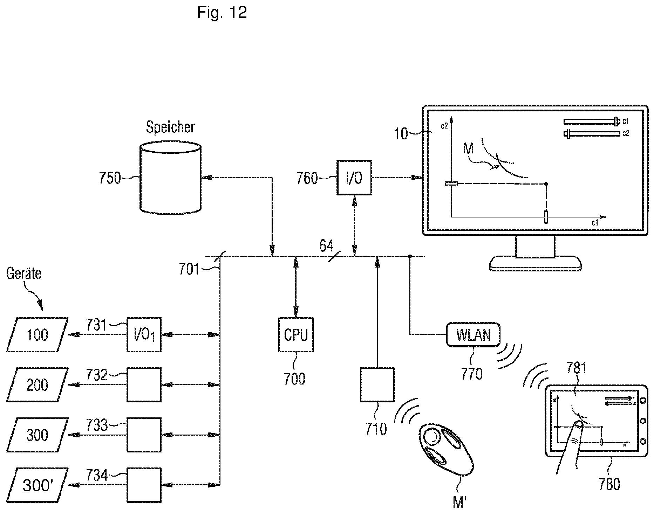

Das erste Strahlengerät 100 der Figur 2A ist ein Strahlengerät 100 zur Abgabe von Protonen während oder für eine Strahlentherapie eines Patienten P, der auf einem Tisch 120 (behandlungs-fertig) positioniert ist. Das erste Strahlengerät steht für die Technologie 100 (oder eine Technologie A). Ein erster Tragkörper 114 ist rotatorisch gelagert und an ihm ist ein Strahlenkopf 110 angeordnet, der über eine Brücke 112 mit dem Tragkörper 114 starr verbunden ist. Über den Tragkörper 114 kann der Winkel des Strahlenkopfes 110 eingestellt werden, der gegenüber dem Patienten P eingenommen wird. Auch die Strahlendosis und die Verteilung der Strahlung innerhalb des nicht dargestellten Protonenstrahls aus dem Strahlenkopf 110 sind einstellbar. Alle diese technischen Werte als Einstellgrößen werden in einer Paretofront repräsentiert, für einen Patienten über eine gesamte fraktionell aufgeteilte Radiotherapie (Strahlentherapie). Im Beispiel kann dies der Paretofront 101 von Figur 1 entsprechen. Die Einstellung der technischen Werte als Einstellgrößen des Strahlenkopfs 110 erfolgt über ein I/O-Interface 731 von dem z.B. 64bit Bus 701 aus, der in Figur 12 ersichtlich ist.

Es soll auch hier erneut darauf hingewiesen werden, dass nicht die Therapie mit dem Strahlengerät 100 (der Technologie 100) beansprucht wird, sondern die Planung dieser Therapie. Der Bezug auf die Therapiegeräte als Strahlengeräte, welche diese Planung später oder funktionell getrennt von der Planung umsetzen, dient der Veranschaulichung für den Patienten, es soll die Offenbarung aber nicht auf die Therapie des Patienten beziehen. Die Planung und die Therapie lassen sich zeitlich und funktional ohne weiteres trennen oder unterscheiden.

Gleiches gilt auch für die folgenden Figuren.

Figur 2B zeigt ein weiteres Strahlengerät, hier ein Photonenstrahlengerät 200 der Technologie 200 (oder eine Technologie B). Der Patient P ist auf einem Tisch 220 platziert und der Strahlenkopf 210 zur Abgabe der Protonen ist an einer L-förmigen Brücke 212 angeordnet. Die Brücke 212 ist rotatorisch mit einem stationären Sockel 214 verbunden, dem gegenüber sie schwenkbar ist. Auch hier können die Einstellungen der Strahlenabgabe des Strahlenkopfes 210 vorgegeben werden, und sie für eine fraktionierte Behandlung einer Paretofront, beispielsweise der Paretofront 101 aus Figur 1, vorgegeben oder eingestellt werden. Die Einstellungen der technischen Werte des Strahlenkopfes 210 erfolgt über ein I/O-Interface 732 von dem z.B. 64bit Bus 701 aus, der in Figur 12 ersichtlich ist.

Figur 2C zeigt ein weiteres Strahlengerät 300 der Technologie 300. Auch hier ist der Patient P auf einem Tisch 320 aufgelegt. Eine Brücke 312 ist schwenkbar an einem Sockel 314 angeordnet und der Strahlenkopf 310 ist ausgebildet, beschleunigte Photonen abzugeben. Die Abgabe der beschleunigten Photonen aus dem Strahlenkopf 310 wird so eingestellt, dass sie auf das Target und die Risiken so abgestimmt sind, dass im Rahmen der fraktionierten Behandlungssitzung primär das Target von den Strahlen in seinem Volumen erfasst wird und die Risiken von einer Strahlenbelastung abgeschirmt werden. Dazu kann ein - nicht dargestellter - Multilamellen-Kollimator vorgesehen sein, der in dem Strahlenkopf 310 platziert ist und dessen Lamellen so eingestellt werden, dass sie in jede beliebige Form gebracht werden, um als freien Raum zwischen sich eine passende Fläche freizugeben, die dem Zielvolumen (Target) bestmöglich entspricht. Die Lamellen selbst sind strahlenabschirmend, beispielsweise aus Wolfram, sodass sich die Form des Strahls nahezu beliebig einstellen lässt. Zusätzlich zu dieser form-gebenden Einstellung des Strahlenvolumens, eigentlich der Strahlenfläche, kann ein Regime treten, mit dem die Felder und die Winkel vorgegeben werden, unter denen die Bestrahlungen erfolgen. Die Einstellung der technischen Werte des Strahlenkopfs 310, z.B. des Multilamellen-Kollimators erfolgt über ein I/O-Interface 733 von dem schon genannten 64bit Bus 701 aus, der in Figur 12 ersichtlich ist.

Figur 2D zeigt ein weiteres Strahlengerät 300', welches demjenigen der Figur 2C entspricht, nur eine ältere oder technisch alte Bauart ist, die dem Therapiegerät D der folgenden Beschreibung entsprechen kann. Die ältere Bauart soll zum Ausdruck bringen, dass es eine eigene Technologie 300' ist, die zwar mit derjenigen des Therapiegerät der Figur 2C übereinstimmt, nur nicht dem aktuellen Stand der technischen Möglichkeiten entspricht. Die hier verwendeten Komponenten entsprechen denen der Figur 2C. Sie sind symbolisch mit einem Strich versehen, also (alte) Brücke 312', (alter) Sockel 314' und (alter) Strahlenkopf 310'. Patient P ist auf dem (alten) Tisch 320' platziert. "Alt" steht für die ältere Technologie 300'. Die Einstellung der technischen Werte des technisch älteren Strahlenkopfs 310', erfolgt über ein I/O-Interface 734 von dem schon genannten 64bit Bus 701 aus, der in Figur 12 ersichtlich ist.

Im Ablauf der Figuren 4A bis 4D entsteht ein Feld F, welches alle Kombinationen an erreichbaren Mischverhältnissen von zwei Technologien mit den Paretofronten 601 und 701 abbildet und dies bei den Zielkriterien c1 und c2.

Anhand der Figur 4E wird die Entstehung des Feldes F erläutert. Zwei Strecken wandern auf den Paretofronten 601 und 701. Eine Strecke 80-90 wird an Punkt 80 festgehalten und bewegt sich von dem Punkt 90 auf der Paretofront 601, bis zum Punkt 92. Die überstrichene Fläche umschreibt eine erste Gruppe von Punkten, welche zum Feld F gehören, hier nicht eingezeichnet. Die zweite Strecke erstreckt sich auch zwischen den Punkten 90 und 80, wobei diese Strecke am Punkt 90 festgehalten wird und auf der Paretofront 701 bis zum Punkt 82 verläuft (sh. dazu Figur 4E). Das ist die zweite Gruppe von Punkten des Feldes F. Gemeinsam bilden sie das Feld F. Deren linker unterer Rand ist die Summe der paretooptimalen Punkte, deren Verbindung die Strecke KK oder 801 ergibt, die Konvexkombination von Punkten.

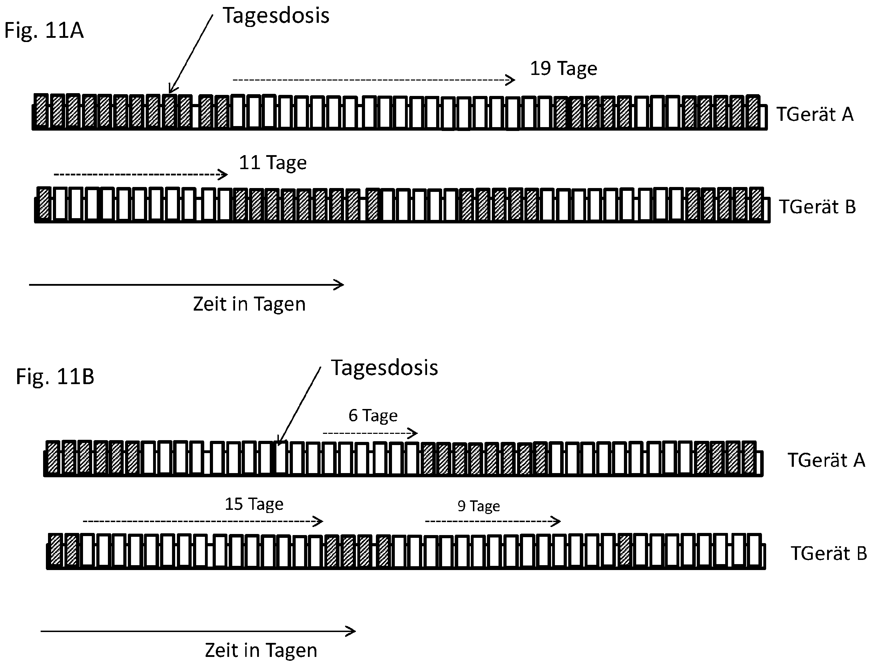

Auf der Strecke 801 der paretooptimalen Punkte kann das Mischungsverhältnis eingestellt, also navigierbar verändert werden (bei der Planung). Zwei Punkte sind hervorgehoben, die sich aus dem durch Verfügbarkeit der Strahlengeräte möglichen Mischungsverhältnis der Figuren 11A und 11B ergeben.

Das ist der durch eine eckige Klammer gekennzeichnete Punkt M2 mit ca. 67% Anteilen der Paretofront 601, die hier für das Strahlengerät A steht. Der durch eine entgegengesetzt ausgerichtete eckige Klammer gekennzeichnete Punkt M4 hat demgegenüber einen Anteil von ca. 67% des Strahlengeräts B. Entsprechend verbleibt für das jeweilige andere Strahlengerät ein Anteil von ca. 33% in der Mischung.

Da die Punkte rechts von M4 näher an der Paretofront 701 liegen, ist hier der Anteil der Paretofront 701 größer. Der Punkt M3 hat einen Anteil von 100% des Strahlengeräts B. Der Punkt M1 liegt auf der Paretofront 601 und hat demgemäß 100% Anteil von dem Strahlengerät A. Ein Punkt entspricht einem Therapieplan, mit seinen technischen Einstellung am Strahlengerät.

Die beiden Punkte M2 und M4 ergaben sich aus der Verfügbarkeit der Therapiegeräte, hier mit Blick auf die Figuren 11A/11B. In der Figur 11A sind die beiden Strahlengeräte TGerät A und TGerät B mit ihren Time Slots von jeweils Tagesdosen aufgetragen. Die Prämisse, schnellstmöglich zu beginnen, führt dazu, dass das Strahlengerät A nicht eingeplant werden kann. Verfügbar ist schnellstmöglich nur das Strahlengerät TGerät B. Dort besteht zur Planung Verfügbarkeit für elf Tagesdosen, bevor dieses Strahlengerät auch belegt ist. Anschließend kann auf das Strahlengerät A gewechselt werden. Dort wird der Rest der 30 Tagesfraktionen mit 19 Tagesdosen eingeplant. Es ergibt sich damit ein Aufteilung von ca. einem Drittel von Strahlengerät B und ca. zwei Drittel von Strahlengerät A. Daraus ergeben sich die beiden durch eckige Klammern markierte "Punkte" M2 und M4 auf der Strecke KK oder 801 in der Figur 4E.

In einem zweiten Beispiel der Figur 11B ist das Strahlengerät A anfangs für fünf Tage belegt, kann also keinen schnellstmöglichen Beginn der zu planenden Therapie ermöglichen. Das Strahlengerät B ist anfangs nur zwei Tage belegt und dann für 15 Tage in der Planung verfügbar. Allerdings ist der dann zu planende Wechsel auf das Therapiegerät A nur für sich anschließende sechs Tage möglich, mit einem erneut anschließend einzuplanenden Wechsel auf das Therapiegerät B, für die verbleibenden neun Tage der auch hier angenommenen Planungsdauer von 30 Tagen für die gesamte fraktionierte Therapie(dauer).

Der erneute Wechsel des Therapiegeräts ist gegebenenfalls medizinisch sinnvoll, aber eigentlich nicht primär erwünscht. Medizinisch sinnvoll könnte er aus zwei Gründen sein
a. Er schont ein spezifisches Organ Z1
b. Er belastet ein anderes Organ Z2, das die Belastung verkraftet.

Erwähnt sein soll, dass der dargestellten Punkt M1 von der Paretofront 601 dominiert wird, bezüglich des Kriteriums c2.

Es wird an der Figur 11B sehr gut erkannt, dass es auch ein Ziel der Planung sein kann, Lücken in der Verfügbarkeit der Strahlengeräte aufzufüllen, unter der Prämisse, dass es medizinisch sinnvoll ist.

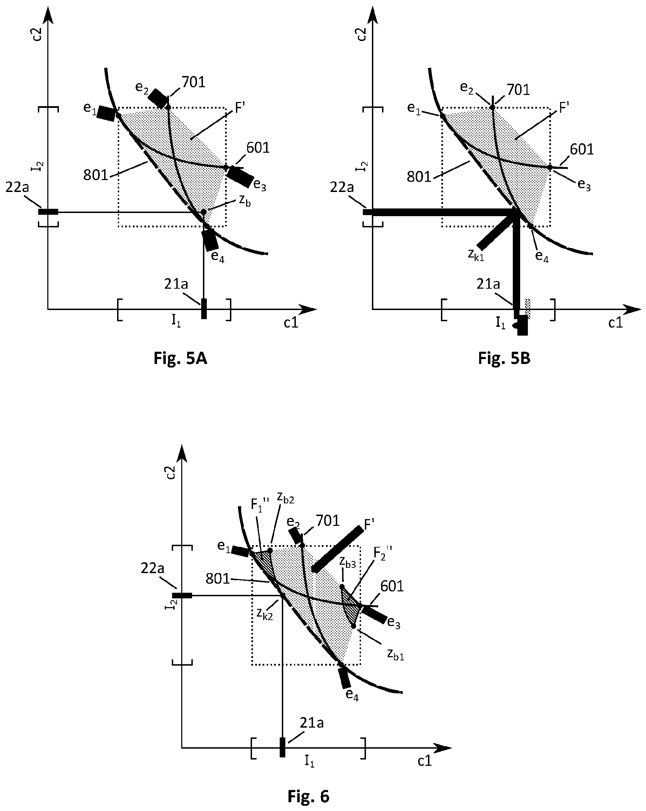

Anhand der Figuren 5A und 5B wird gezeigt, dass die Navigation auf der Strecke 801 (die Summe der paretooptimalen Punkte) auch mit den Slidern 21a und 22a der Bedienhilfen 21 und 22 erfolgen kann, die in Figur 1 gezeigt sind.

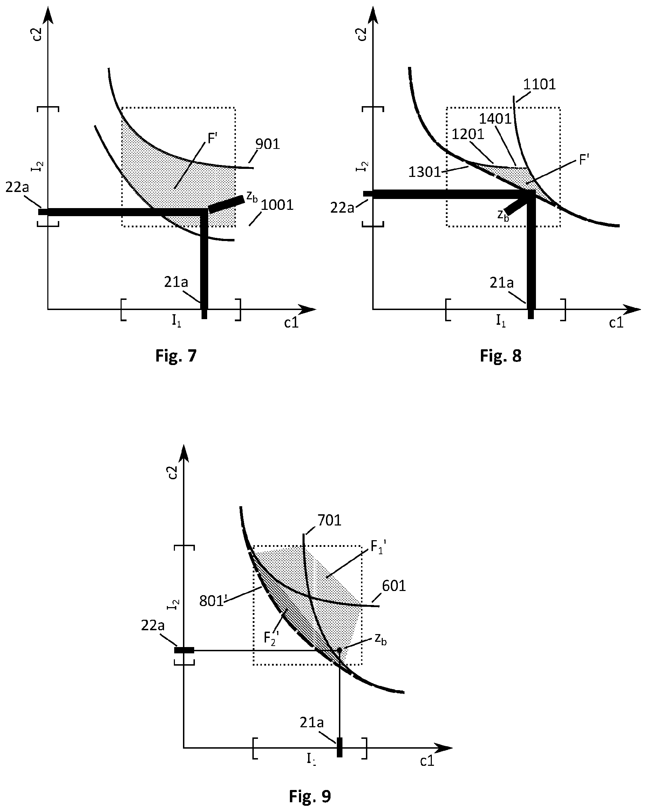

Figur 7 veranschaulicht ein Feld F' zwischen zwei Technologien 901 und 1001, dabei ist eine der Technologien strikt besser als die andere. Therapien, die näher an der Funktion B als Repräsentant des Strahlengeräts B liegen, können früher begonnen werden, als diejenigen Planungen von Therapien die näher an der Funktion A liegen.

Eine andere Möglichkeit der Lage der Paretofronten der Strahlengeräte A und B, entsprechend 601 und 701, ist diejenige, dass eine Lücke 1401 im therapeutischen Fenster des Kriteriums c1 besteht, die durch die Strecke 1301 mit einem Mischungsverhältnis der beiden zu planenden Therapien überbrückt werden kann (in der Planung).

Ergeben sich aus Figur 9 Synergieeffekte, ergänzt die Strecke 801' einen weiteren bauchigen Anteil F2' zu der gemeinsamen Fläche F (zusätzlich zur vorhandenen Fläche F1').

Figur 12 zeigt eine netzwerkorientierte Darstellung der Steuerung über den bereits erwähnten Bus 701 und die daran angekoppelten und verwendeten digitalen Komponenten. Der Bus 701 ist im bevorzugten Beispiel einen 64bit Bus, über den die digitalen Komponenten miteinander kommunizieren. Er ist bidirektional. Für die Rechenkapazität und die Rechenfunktion ist die CPU 700 vorgesehen, welche sich die auf dem Display 10 dargestellten Funktionen 401 und 101 aus dem Speicher 750 ausliest, und sie der Anzeigeeinheit I/O 760 über den Bus 701 weiterleitet. In der Anzeigeeinheit 760 werden die Daten je nach technischem Typ des Displays 10 daten-technisch aufbereitet und sichtbar dargestellt.

Dargestellt ist die Navigationsumgebung der Figur 1. Sie wird über den Funktionszeiger M bedient und er wird vom Planer über ein Mausgerät, hier im Beispiel als Trackball M', bewegt und aktiviert (Anwahl einer Funktion an einem Ort des Zeigens des Zeigers M). Der Trackball M' ist per Funk mit einem Empfänger 710 gekoppelt, der seine Bewegungssignale umsetzt und an den Bus 701 weiterleitet. Diese Kopplung ist nicht bidirektional.

Dem Display 10 ist das Mausgerät M' zugeordnet, bspw. als der Trackball, der die Funktionen der Zeigens und des Bedienens (das Auslösen einer Funktion) ermöglicht, dies am Ort der Anzeige des Mauszeigers M.

Mit dem Mauszeiger M werden ebenso beide Bedienhilfen 21 und 22 für die Kriterien c1 und c2 bedient.

Ein alternatives Display 781 ist das eines Tablets 780, welches mit der gleichen Darstellung der Figur 1 versehen ist, welche auf dem touch-sensitiven Display 781 dargestellt wird. Die Bildschirmanzeige oder die Tablet-Anzeige hat einen Patchbereich 1 und den Bedienbereich 2, wie zuvor erläutert. Das Tablet 780 ist über eine WLAN-Kopplung (oder Anbindung) mit einem WLAN-Sender und Empfänger 770 an dem Bus 701 angekoppelt. Der Mauszeiger wird durch den Finger (wie dargestellt) ersetzt und durch Gesten (z.B. Swipe, Tap, double Tap) auf dem Display 781 des screen-enabled oder touch-sensitiven Tablet 780 gesteuert.

Die technologischen Strahlengeräte 100 bis 300' sind über jeweils ein Input/Output Device 731, 732, 733 und 734 an den Bus 701 angekoppelt. Jedes dieser Input/Output Devices ist bidirektional, kann also einstellende Parameter von dem Bus 701 auf das jeweilige Strahlengerät vorgeben.

Bevorzugt hat jedes dieser Strahlengeräte 100, 200, ... einen ausreichenden Speicherplatz von zumindest 500GB, so dass es die vor-eingestellten Parameter der späteren Therapie speichern und während der Therapie eigenständig, also autark einstellen kann, besonders für fraktionierte Sitzungen der Patienten über den Planungszeitraum.

Im Beispiel sendet I/O₁ die Einstellparameter an das Strahlengerät 100 zur Abgabe von Protonen. Dieses ist dann in der Lage, die einzustellenden, eigentlich die dann bereits vorgegebenen Parameter des Strahlengeräts für die Technologie 100 "Protonen" während der über Tage verteilten (fraktionierten) Therapie des Patienten P anzuwenden. Also die Dosen und Intensitäten und Richtungen an Protonenstrahlungen (in der Radiotherapie) an diesen individuellen Patienten P abzugeben. Hier erfolgt die funktionelle Trennung von Planung und Therapie.

Dieses Beispiel ist auch so steuerbar, dass die Parameter im Speicher 750 über die Dauer der fraktionierten Therapie vorgehalten werden, und nur zu den Zeiten vor der jeweiligen Therapiesitzung an das Strahlengerät 100 übertragen werden. Diese fraktionierte Therapie ist dabei eine fraktionierte Programmierung des jeweiligen Strahlengeräts 100 bis 300' (im Sinne der fraktionierten Datenübertragung des jeweils aktuell benötigten zeitlichen Abschnitts der Therapie von z.B. 30 Tagen). Die eigentliche Therapie führt das Strahlengerät 100 selbsttätig, und ohne die schon zuvor erfolgte und fertige Planung durch.

In gleicher Weise werden die anderen Geräte 200, 300 und 300' programmiert, für die Therapie vorbereitet und datentechnisch konditioniert.

Es ist hier erneut zu betonen, dass keine Therapie gleichzeitig mit der Planung erfolgt, die Therapie ist bereits fertig geplant und funktionell vorbereitet (also fertig geplant), bevor das jeweilige Gerät diese Radiotherapie am Patienten ausführt. Letztere Therapie ist nicht beansprucht.

Eine Navigation über die dargestellten Patches auf dem Display 10 kann erst vom Planer abgeschlossen werden, um sie später in das oder die zugehörigen Strahlengeräte über das jeweilige der Input/Output Devices 731 bis 734 zu übertragen.

## Patentansprüche

1. Verfahren zum Entwerfen oder Gestalten einer Therapie als Behandlungsplan, vor einer Behandlung und mit einer interaktiven Navigation an einem Sichtgerät (10)
(a) wobei zumindest zwei Technologien (A, B) zumindest zweier Strahlengeräte (100,200,300,300') auf einem Sichtgerät (10) dargestellt werden und zur Auswahl stehen, unter Bereitstellung zumindest einer Technologie (A) eines ersten Strahlengeräts (100) und zumindest einer Technologie (B) eines zweiten Strahlengeräts (200);
(b) für eine nach dem Entwerfen oder Gestalten zu behandelnde Person (P) mit einer therapierbaren Erkrankung, die von nicht gleichzeitigen fraktionierten Dosen der zumindest zwei Strahlengeräte (100,200) therapiert wird, wobei der entworfene oder gestaltete Behandlungsplan eine Vielzahl von technischen Einstellungen definiert, welche an den Strahlengeräten vor der Behandlung eingestellt werden;
**dadurch gekennzeichnet, dass**
(c1) auf dem Sichtgerät (10) für die zumindest zwei Technologien (A,B) je eine Paretofront (601,701) über jeweils zumindest zwei Kriterien (c1,c2) dargestellt werden;
(c2) eine kombinierte Paretofront (801) aus einem Feld (F,F') der beiden Kriterien (c1,c2) gebildet wird, entstanden aus Konvexkombinationen von zumindest Stützstellen der ersten und zweiten Paretofront (601,701);
(c3) ein Flächenabschnitt als Feld (F, F') gebildet wird;
so dass vom Feld (F, F') eine Strecke begrenzter Länge gebildet wird, welche die kombinierte Paretofront (801) bildet, zur interaktiven Navigation eines Mischungsverhältnisses aus zumindest zwei Technologien (A, B) verfügbar gemacht wird und zur Planung benutzt wird, zum Entwerfen oder Gestalten des Behandlungsplans als einen Punkt auf der kombinierten Paretofront (801).

2. Verfahren nach Anspruch 1, wobei auch interpolierte Zwischenwerte der Stützstellen durch die Konvexkombination in neuen Punkten neben den Paretofronten abgebildet werden.

3. Verfahren nach Anspruch 1 oder 2, wobei die Strecke als kombinierte Paretofront (801) die Paretofronten (601,701) nicht schneidet.

4. Verfahren nach einem der vorherigen Ansprüche, wobei die per Konvexkombinationen neben den Paretofronten (601,701) gebildeten neuen Punkte das Feld (F,F') im therapeutischen Fenster (f) aufspannen.

5. Verfahren nach einem der vorherigen Ansprüche, wobei der Planungspunkt auf der kombinierten Paretofront (801) fraktionierte Anteile der Technologie (A) des ersten Strahlengeräts (100) und fraktionierte Anteile der Technologie (B) eines zweiten Strahlengeräts (200) aufweist.

6. Verfahren nach beiden voriger Ansprüche, wobei Punktemengen zur Bildung eines Flächenabschnitts zusammengenommen werden, und diese ins Verhältnis gesetzt werden, das von einer zeitlichen Verfügbarkeit der zumindest zwei Strahlengeräte (100,200,300,300') begrenzt wird.

7. Verwenden eines Sichtgeräts (10) für ein Entwerfen oder Gestalten einer Therapie als Behandlungsplan, vor einer Behandlung und mit einer interaktiven Navigation an dem Sichtgerät (10) unter Verwendung der kombinierten Paretofront (801) nach einem der Ansprüche 1 bis 6.

8. Verwendung nach Anspruch 7, ausgebildet für ein Entwerfen oder Gestalten des Behandlungsplans als einen Punkt auf der kombinierten Paretofront (801).

## Claims

1. Method for designing or creating a therapy as a treatment plan, prior to treatment and with interactive navigation on a display device (10)
(a) wherein at least two technologies (A, B) of at least two radiation devices (100, 200, 300, 300') are displayed on a display device (10) and are available for selection, providing at least one technology (A) of a first radiation device (100) and at least one technology (B) of a second radiation device (200);
(b) for a person (P) to be treated according to the designed or configurate , who has a treatable disease that is treated by non-concurrent fractionated doses from the at least two radiation devices (100, 200), wherein the designed or configured treatment plan defines a plurality of technical settings that are set on the radiation devices prior to treatment;
**characterised in that**
(c1) on the display device (10) for the at least two technologies (A, B), a Pareto front (601, 701) is displayed for each of at least two criteria (c1, c2);
(c2) a combined Pareto front (801) is formed from a field (F, F') of the two criteria (c1, c2), arising from convex combinations of at least support points of the first and second Pareto fronts (601, 701);
(c3) an area section is formed as a field (F, F');
so that a line of limited length is formed from the field (F, F'), which forms the combined Pareto front (801), is made available for interactive navigation of a mixture ratio of at least two technologies (A, B) and is used for planning, for designing or creating the treatment plan as a point on the combined Pareto front (801).

2. Method according to claim 1, wherein interpolated intermediate values of the support points are also mapped by the convex combination at new points adjacent to the Pareto fronts.

3. Method according to claim 1 or 2, wherein the line as a combined Pareto front (801) does not intersect the Pareto fronts (601, 701).

4. Method according to one of the previous claims, wherein the new points formed by convex combinations adjacent to the Pareto fronts (601, 701) span the field (F, F') in the therapeutic window (f).

5. Method according to one of the previous claims, wherein the planning point on the combined Pareto front (801) has fractional proportions of the technology (A) of the first radiation device (100) and fractional proportions of the technology (B) of a second radiation device (200).

6. Method according to both of the preceding claims, wherein sets of points are combined to form an area section, and these are put into a relationship that is limited by the temporal availability of the at least two radiation devices (100, 200, 300, 300').

7. Using a display device (10) for designing or planning a therapy as a treatment plan, prior to treatment and with interactive navigation on the display device (10) using the combined Pareto front (801) according to one of claims 1 to 6.

8. Use according to claim 7, designed for designing or creating the treatment plan as a point on the combined Pareto front (801).

## Revendications

1. Procédé pour concevoir ou élaborer une thérapie sous forme de plan de traitement, avant un traitement et avec une navigation interactive sur un dispositif d'affichage (10)
(a) au moins deux technologies (A, B) d'au moins deux appareils à rayonnement (100, 200, 300, 300') étant représentées sur un dispositif d'affichage (10) et pouvant être sélectionnées, en mettant à disposition au moins une technologie (A) d'un premier appareil à rayonnement (100) et au moins une technologie (B) d'un deuxième appareil à rayonnement (200);
(b) pour une personne (P) à traiter après la conception ou l'élaboration d'un plan de traitement ( ) ( ) et souffrant d'une maladie pouvant être traitée, qui est traitée par des doses fractionnées non simultanées provenant d'au moins deux appareils à rayonnement (100, 200), le plan de traitement conçu ou élaboré définissant une pluralité de réglages techniques qui sont réglés sur les appareils à rayonnement avant le traitement;
**caractérisé en ce que**
(c1) sur le dispositif d'affichage (10), pour les au moins deux technologies (A, B), un front de Pareto (601, 701) est représenté pour chacune d'elles sur la base d'au moins deux critères (c1, c2);
(c2) un front de Pareto combiné (801) est formé à partir d'un champ (F, F') des deux critères (c1, c2), résultant de combinaisons convexes d'au moins des points d'appui des premier et deuxième fronts de Pareto (601, 701);
(c3) une section de surface est formée en tant que champ (F, F');
de sorte qu'à partir du champ (F, F'), un segment de longueur limitée est formé, qui constitue le front de Pareto combiné (801), est mis à disposition pour la navigation interactive d'un rapport de mélange d'au moins deux technologies (A, B) et est utilisé pour la planification, pour concevoir ou élaborer le plan de traitement comme un point sur le front de Pareto combiné (801).

2. Procédé selon la revendication 1, dans lequel des valeurs intermédiaires interpolées des points d'appui sont également représentées par la combinaison convexe en de nouveaux points à côté des fronts de Pareto.

3. Procédé selon la revendication 1 ou 2, dans lequel la ligne en tant que front de Pareto combiné (801) ne coupe pas les fronts de Pareto (601, 701).

4. Procédé selon l'une des revendications précédentes, dans lequel les nouveaux points formés par des combinaisons convexes à côté des fronts de Pareto (601, 701) couvrent le champ (F, F') dans la fenêtre thérapeutique (f).

5. Procédé selon l'une des revendications précédentes, dans lequel le point de planification sur le front de Pareto combiné (801) présente des parts fractionnées de la technologie (A) du premier appareil à rayonnement (100) et des parts fractionnées de la technologie (B) d'un deuxième appareil à rayonnement (200).

6. Procédé selon les deux revendications précédentes, dans lequel des ensembles de points sont combinés pour former une section de surface, et ceux-ci sont mis en relation, qui est limitée par une disponibilité temporelle d'au moins deux appareils à rayonnement (100, 200, 300, 300').

7. Utilisation d'un dispositif d'affichage (10) pour concevoir ou élaborer une thérapie sous forme de plan de traitement, avant un traitement et avec une navigation interactive sur le dispositif d'affichage (10) en utilisant le front de Pareto combiné (801) selon l'une des revendications 1 à 6.

8. Utilisation selon la revendication 7, conçue pour concevoir ou élaborer le plan de traitement comme un point sur le front de Pareto combiné (801).
